# EUROPÄISCHE PATENTSCHRIFT

(11) **EP 0 799 213 B1**
(45) Veröffentlichungstag und Bekanntmachung des Hinweises auf die Patenterteilung: **18.11.1998**
(21) Anmeldenummer: 95942138.9
(22) Anmeldetag: 13.12.1995
(51) Int. Cl.: C07D 251/62

(54) **VERFAHREN ZUR REINIGUNG VON MELAMIN**
MELAMINE PURIFYING PROCESS
PROCEDE DE PURIFICATION DE MELAMINE

(30) Priorität: 23.12.1994 AT 2393/94
(43) Veröffentlichungstag der Anmeldung: 08.10.1997
(73) Patentinhaber: Agrolinz Melamin GmbH, A-4021 Linz (AT)
(72) Erfinder: FINGRHUT, Helmut, A-4020 Linz (AT); CANZI, Lorenzo, I-20131 Mailand (IT); COUFAL, Gerhard, I-22070 Appiano Gentile (IT); GIACOMUZZO, Silvano, I-21012 Cassano Magnago (IT); MÜLLNER, Martin, A-4020 Linz (AT)
(74) Vertreter: Kunz, Ekkehard, Dr.
(86) Internationale Anmeldenummer: EP9504921
(87) Internationale Veröffentlichungsnummer: WO9620183

(56) Entgegenhaltungen:
- GB-A- 1 032 326
- US-A- 3 116 294
- 'Ullman's Encyclopedia of Industrial Chemistry, Vol. A16, Edition 5' , VCH VERLAG , 1991 in der Anmeldung erwähnt siehe Seite 173

## Beschreibung

Aus der Literatur ist bereits eine Vielzahl von Verfahren zur Herstellung von Melamin bekannt. Ein bevorzugtes Ausgangsmaterial ist dabei Harnstoff, der entweder bei hohem Druck und nichtkatalytisch oder bei niedrigem Druck und unter Verwendung eines Katalysators zu Melamin, Ammoniak und CO₂ umgesetzt wird. Eines dieser Verfahren ist das in US-A-3,116,294 beschriebene Hochdruckverfahren, nach welchem das flüssige Melamin nach Abtrennen der CO₂ und NH₃-Abgase, nach Behandeln mit NH₃ im Gegenstrom eine bestimmte Zeit unter Ammoniakdruck verweilen gelassen wird, worauf die Schmelze durch Abschrecken mit Wasser oder Mischen mit kalten Gasen rasch abgekühlt wird. Es ist auch bekannt, daß bei diesen Verfahren, insbesondere bei den Hochdruckverfahren, verschiedene Nebenprodukte bzw. Verunreinigungen, wie beispielsweise Melem, Melam, Ammelin, Ammelid oder Ureidomelamin, entstehen, die die Reinheit des Melamins beeinträchtigen.
Bisher übliche Reinigungsverfahren, wie etwa das Umkristallisieren, sind jedoch mit Nachteilen behaftet. So ergibt sich besonders bei der Umkristallisation der Nachteil, daß sich die Verunreinigungen in der Mutterlauge anreichern und deshalb ein beträchtlicher Teil derselben, die auch Melamin enthält, verworfen oder nachbehandelt werden muß. Weiters ist in CH 345.894 beschrieben, daß Verunreinigungen wie Melem, Melam oder Ureidomelamin, in Wasser und wäßrigen Alkalilösungen unlöslich, bzw. schwer löslich sind, sodaß diese Verbindungen durch Umkristallisation nur schwer und mit Melaminverlusten aus dem zu reinigenden Melamin entfernt werden können.

Aufgabe der vorliegenden Erfindung war es daher, ein Verfahren zu finden, bei welchem die in Wasser oder in wäßrigen Alkalilösungen unlöslichen oder schwer löslichen Verunreinigungen, insbesondere Melam und Ureidomelamin, in dem zu reinigenden Melamin unter Vermeidung großer Melaminverluste deutlich reduziert werden können.

Unerwarteterweise konnte diese Aufgabe durch ein Verfahren gelöst werden, bei welchem verunreinigtes Melamin eine gewisse Zeit in Anwesenheit von Ammoniak bei erhöhter Temperatur, die unterhalb des Schmelzpunktes von Melamin liegt, gehalten wird.

Gegenstand der vorliegenden Erfindung ist demnach ein Verfahren zur Reinigung von Melamin, das dadurch gekennzeichnet ist, daß verunreinigtes Melamin auf eine Temperatur, die über 200°C und unter dem Schmelzpunkt von Melamin liegt, gebracht und bei einem Ammoniakdruck von 5 bis 350 bar für eine Dauer von 1 Minute bis zu 20 Stunden in diesem Temperaturbereich gehalten wird, worauf in beliebiger Reihenfolge entspannt und auf Raumtemperatur abgekühlt und reines Melamin in Pulverform erhalten wird.

Das erfindungsgemäße Verfahren eignet sich zur Reinigung von Melamin, das in einem beliebigen aus dem Stand der Technik bekannten Prozeß anfällt und insbesondere Verunreinigungen wie Melam und Ureidomelamin enthält. Es wurde gefunden, daß insbesondere der Gehalt an Melam und Ureidomelamin dadurch reduziert werden kann, daß verunreinigtes Melamin, das in kristalliner Form oder als Pulver vorliegt, erwärmt wird und in Anwesenheit von Ammoniak für eine bestimmte Zeit in einem Temperaturbereich unterhalb des Schmelzpunktes von Melamin gehalten wird (Tempern). Es ist aber auch möglich, Melamin, das eine Temperatur aufweist, die über diesem Bereich liegt, auf diesen Temperaturbereich abzukühlen. Der Temperaturbereich, in dem das Tempern durchgeführt wird, liegt dabei über 200°C und unterhalb des Schmelzpunktes von Melamin. Bevorzugt liegt der Temperaturbereich zwischen 240°C, besonders bevorzugt zwischen 250°C und unterhalb des Schmelzpunktes von Melamin. Das Melamin wird dabei auf eine Anfangstemperatur, die in diesem Bereich liegt, gebracht. Die Temperatur kann während des Temperns konstant gehalten werden, sie kann jedoch auch innerhalb des oben definierten Temperaturbereiches verändert werden. So kann die Temperatur beispielsweise kontinuierlich oder diskontinuierlich innerhalb der Grenzen des Temperaturbereiches abgesenkt bzw. erhöht werden.
Die Zeit, während der das Melamin in diesem Temperaturbereich gehalten wird, kann in einem weiten Bereich variieren. Sie hängt hauptsächlich vom gewünschten Endwert an Melam und Ureidomelamin, sowie vom jeweiligen Ammoniakdruck und von wirtschaftlichen Faktoren ab und liegt zwischen 1 Minute und 20 Stunden, bevorzugt zwischen 10 Minuten und 10 Stunden, besonders bevorzugt zwischen 0,5 und 3 Stunden. Der Ammoniakdruck kann ebenfalls in einem großen Bereich variieren und liegt zwischen 5 und 350 bar. Bevorzugt wird ein Druck zwischen 10 und 100 bar eingestellt.
Nach Beendigung des Temperns kann, je nach den technischen Gegebenheiten, zuerst abgekühlt und dann entspannt, oder in umgekehrter Reihenfolge zuerst entspannt und danach abgekühlt werden. Die Durchführung dieser Schritte kann gegebenenfalls in einer weiteren Reaktionsapparatur erfolgen. Melamin wird dabei auf Raumtemperatur, beispielsweise mit Hilfe von Wärmetauschern, einfaches Entfernen des Heizmediums und Stehenlassen des Mediums oder durch Mischen mit kalten Gasen, abgekühlt. Melamin wird durch das erfindungsgemäße Verfahren in kristalliner Form bzw. als Pulver erhalten und weist insbesondere einen deutlich reduzierten Gehalt an Melam und Ureidomelamin auf, der teilweise sogar unter der Nachweisgrenze liegt.
Das erfindungsgemäße Verfahren kann sowohl diskontinuierlich als auch kontinuierlich durchgeführt werden.
Das erfindungsgemäße Verfahren kann auch an jeden beliebigen, aus dem Stand der Technik bekannten Melaminprozeß gekoppelt werden. In einer bevorzugten Ausführungsform wird das erfindungsgemäße Verfahren mit einem der aus dem Stand der Technik bekannten Hochdruckprozesse, wie etwa in Ullmann's Encyclopedia of Industrial Chemistry, 5^{th} Edition, Vol. A-16, pp 174-179 beschrieben, kombiniert. Besonders bevorzugt wird das erfindungsgemäße Verfahren im Anschluß an bereits bekannte Aufarbeitungsschritte der Hochdruckverfahren durchgeführt. Diese Aufarbeitungsschritte beinhalten
a) Abtrennen des bei der Umsetzung von Harnstoff erhaltenen NH₃/CO₂-Gasgemisches vom flüssigen Melamin,
b) Reduktion des im Melamin gelösten CO₂ durch Einbringen von NH₃, sowie
c) Verweilenlassen des flüssigen Melamins in Anwesenheit von Ammoniak und
d) Abkühlen des Melamins beispielsweise durch Abschrecken mit kaltem Wasser oder flüssigem Ammoniak oder durch Mischen mit kaltem Gas.
Diese Aufarbeitungsschritte sind beispielsweise in US 3,116,294 beschrieben.

Das erfindungsgemäße Verfahren wird dabei im Anschluß an Schritt d) durchgeführt, wobei die Anzahl der durchgeführten vorangegangenen Aufarbeitungsschritte a) bis c) nach den jeweiligen Gegebenheiten variieren kann. Die Aufarbeitung des durch ein Hochdruckverfahren aus Harnstoff gewonnenen Melamins bis zur Abkühlphase d) kann somit die Schritte a) bis c), sowie jede beliebige Zweier-Kombination oder auch nur einen dieser Schritte umfassen.

### Beispiel 1-20:

In einem Miniautoklaven mit 10 ml Volumen wurde eine bestimmte Menge (EW) an Melamin mit einem Ausgangsgehalt von 18300 ppm Melam und 10 600 ppm Ureidomelamin, sowie die zur Aufrechterhaltung eines bestimmten Druckes p nötige Ammoniakmenge eingebracht. Anschließend wurde der Autoklav auf eine Temperatur T gebracht und t Minuten auf dieser Temperatur gehalten. Danach wurde der Autoklav rasch durch Eintauchen in kaltes Wasser abgekühlt und dann entspannt. Das so gereinigte Melamin wurde auf den Gehalt an Melam und Ureidomelamin untersucht, wobei der Gehalt an Ureidomelamin stets unter der Nachweisgrenze von 100 ppm lag. Die Verfahrensparameter wie, Einwaage an Melamin (EW), Druck p, Zeit t, Temperatur T, Endgehalt an Melam (MA) sind aus Tabelle 1 ersichtlich.

**Tabelle 1**

| Bsp. | EW (mg) | p (bar) | T (°C) | t (min) | MA (ppm) |
|---|---|---|---|---|---|
| 1 | 22,03 | 10 | 250 | 10 | 5000 |
| 2 | 22,03 | 10 | 250 | 100 | 3800 |
| 3 | 22,03 | 10 | 250 | 1000 | 1100 |
| 4 | 67,93 | 30 | 250 | 10 | 3000 |
| 5 | 67,93 | 30 | 250 | 100 | 2500 |
| 6 | 67,93 | 30 | 250 | 1000 | 820 |
| 7 | 21,19 | 10 | 270 | 10 | 6100 |
| 8 | 21,19 | 10 | 270 | 100 | 4500 |
| 9 | 21,19 | 10 | 270 | 1000 | 190 |
| 10 | 65,16 | 30 | 270 | 10 | 3000 |
| 11 | 65,16 | 30 | 270 | 100 | 2500 |
| 12 | 65,16 | 30 | 270 | 1000 | 280 |
| 13 | 20,42 | 10 | 290 | 10 | 3100 |
| 14 | 20,42 | 10 | 290 | 100 | 1900 |
| 15 | 20,42 | 10 | 290 | 1000 | 250 |
| 16 | 62,61 | 30 | 290 | 10 | 3700 |
| 17 | 62,61 | 30 | 290 | 100 | 1000 |
| 18 | 62,61 | 30 | 290 | 1000 | 120 |
| 19 | 286,57 | 350 | 290 | 10 | 320 |
| 20 | 280,57 | 350 | 290 | 100 | 40 |

### Beispiel 21 - 26:

In einem Autoklaven mit 2 1 Volumen wurde eine bestimmte Menge (EW) an Melamin mit einem Ausgangsgehalt von MA₀ an Melam und UM₀ an Ureidomelamin, sowie die zur Aufrechterhaltung eines bestimmten Druckes p nötige Ammoniakmenge eingebracht. Anschließend wurde der Autoklav auf eine Temperatur T gebracht und t Minuten auf dieser Temperatur gehalten. Danach wurde der Autoklav abgekühlt und dann entspannt. Das so gereinigte Melamin wurde auf den Gehalt an Melam (MA) und Ureidomelamin (UM) untersucht. die Verfahrensparameter wie, Einwaage an Melamin (EW), Druck p, Zeit t, Temperatur T, Anfangsgehalt an Melam (MA₀) und an Ureidomelamin (UM₀) und Endgehalt an Melam (MA) und an Ureidomelamin (UM) sind aus Tabelle 2 ersichtlich.

**Tabelle 2:**

| | | | | | Anfang | | Ende | |
|---|---|---|---|---|---|---|---|---|
| Bspl. | EW (g) | p (bar) | T (°C) | t (h) | MA₀ | UM₀ | MA | UM |
| 21 | 300 | 20 | 250 | 2 | 1200 | 600 | < 300 | < 50 |
| 22 | 300 | 40 | 300 | 1,5 | 1200 | 600 | < 300 | < 50 |
| 23 | 300 | 56 | 250 | 2 | 1200 | 600 | < 300 | < 50 |
| 24 | 300 | 70 | 300 | 4 | 1200 | 600 | < 300 | < 50 |
| 25 | 300 | 125 | 296 | 3 | 1200 | 1300 | < 300 | < 50 |
| 26 | 51,1 | 139 | 250 | 3 | 2500 | < 500 | < 300 | < 50 |

## Patentansprüche

1. Verfahren zur Reinigung von Melamin, dadurch gekennzeichnet, daß verunreinigtes Melamin auf eine Temperatur, die über 200°C und unterhalb des vom jeweilig herrschenden Ammoniakdruckes abhängigen Schmelzpunktes von Melamin liegt, gebracht und bei einem Ammoniakdruck von 5 bis 350 bar für eine Dauer von 1 Minute bis zu 20 Stunden in diesem Temperaturbereich gehalten wird, wobei das Melamin als Feststoff vorliegt, worauf in beliebiger Reihenfolge entspannt und auf Raumtemperatur abgekühlt und reines Melamin in Pulverform erhalten wird.

2. Verfahren nach Anspruch 1, dadurch gekennzeichnet, daß das feste Melamin in einem Temperaturbereich zwischen 240°C und unterhalb des vom jeweilig herrschenden Ammoniakdruckes abhängigen Schmelzpunktes von Melamin gehalten wird.

3. Verfahren nach Anspruch 1, dadurch gekennzeichnet, daß der Ammoniakdruck zwischen 10 und 100 bar liegt.

4. Verfahren nach Anspruch 1, dadurch gekennzeichnet, daß das Verfahren an einen Prozeß zur Hestellung von Melamin angeschlossen wird.

5. Verfahren nach Anspruch 1, dadurch gekennzeichnet, daß das Verfahren an einen Hochdruckprozeß zur Herstellung von Melamin aus Harnstoff angeschlossen wird.

## Claims

1. Process for the purification of melamine, characterized in that contaminated melamine is brought to a temperature which is above 200°C and below the melting point of melamine which is dependent on the respectively prevailing ammonia pressure and is held in this temperature range, where the melamine is presented as a solid, for a period of 1 minute up to 20 hours at an ammonia pressure of 5 to 350 bar, whereupon, in any desired sequence, the reaction mixture is depressurized and cooled to room temperature and pure melamine is obtained in powder form.

2. Process according to Claim 1, characterized in that the solid melamine is held within a temperature range between 240°C and below the melting point of melamine which is dependent on the respectively prevailing ammonia pressure.

3. Process according to Claim 1, characterized in that the ammonia pressure is between 10 and 100 bar.

4. Process according to Claim 1, characterized in that the process follows a process for the production of melamine.

5. Process according to Claim 1, characterized in that the process follows a high-pressure process for the production of melamine from urea.

## Revendications

1. Procédé de purification de mélamine, caractérisé en ce qu'on amène de la mélamine contenant des impuretés à une température supérieure à 200°C et inférieure au point de fusion de la mélamine, qui dépend de la pression d'ammoniac ambiante respective, et on la maintient dans cette plage de températures pendant une durée de 1 minute à 20 heures et sous une pression d'ammoniac de 5 à 350 bars, la mélamine étant à l'état solide, après quoi, dans un ordre quelconque, on relâche la pression et on effectue un refroidissement à la température ambiante et on obtient de la mélamine pure à l'état pulvérulent.

2. Procédé selon la revendication 1, caractérisé en ce qu'on maintient la mélamine solide dans une plage de températures comprise entre 240°C et une température inférieure au point de fusion de la mélamine, qui dépend de la pression d'ammoniac ambiante respective.

3. Procédé selon la revendication 1, caractérisé en ce que la pression d'ammoniac est comprise entre 10 et 100 bars.

4. Procédé selon la revendication 1, caractérisé en ce qu'on le met en oeuvre à la suite d'un procédé de préparation de mélamine.

5. Procédé selon la revendication 1, caractérisé en ce qu'on le met en oeuvre à la suite d'un procédé à haute pression pour la préparation de mélamine à partir d'urée.
